(19) Europäisches Patentamt
European Patent Office
Office européen des brevets

(11) Veröffentlichungsnummer : **0 024 030**
**B1**

(12) # EUROPÄISCHE PATENTSCHRIFT

(45) Veröffentlichungstag der Patentschrift : **30.03.83**

(51) Int. Cl.$^3$ : **C 07 D207/27**, C 07 D207/273, A 61 K 31/40

(21) Anmeldenummer : **80104604.6**

(22) Anmeldetag : **05.08.80**

(54) **Pyrrolidin-Derivate, Verfahren zu deren Herstellung und diese enthaltende Arzneimittel.**

(30) Priorität : **09.08.79 CH 7308/79**
**20.06.80 CH 4755/80**

(43) Veröffentlichungstag der Anmeldung : **18.02.81 Patentblatt 81/07**

(45) Bekanntmachung des Hinweises auf die Patenterteilung : **30.03.83 Patentblatt 83/13**

(84) Benannte Vertragsstaaten :
**AT BE CH DE FR IT LI LU NL SE**

(56) Entgegenhaltungen :
**DE A 2 413 935**
**DE A 2 541 855**
**Chemical Abstracts 63, 16256 d (1965)**

(73) Patentinhaber : **F. HOFFMANN-LA ROCHE & CO. Aktiengesellschaft**
**CH-4002 Basel (CH)**

(72) Erfinder : **Aschwanden, Werner**
**Grellingerstrasse 4**
**CH-4107 Ettingen (CH)**
Erfinder : **Kyburz, Emilio, Dr.**
**Unterer Rebbergweg 127**
**CH-4153 Reinach (CH)**

(74) Vertreter : **Lederer, Franz, Dr. et al**
**Patentanwälte Lederer, Meyer Lucile-Grahn-Strasse 22**
**D-8000 München 80 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 024 030 B1

Pyrrolidin-Derivate, Verfahren zu deren Herstellung und diese enthaltende Arzneimittel

Die vorliegende Erfindung betrifft Pyrrolidin-Derivate. Im Speziellen betrifft sie 1-substituierte 2-Pyrrolidinone der allgemeinen Formel

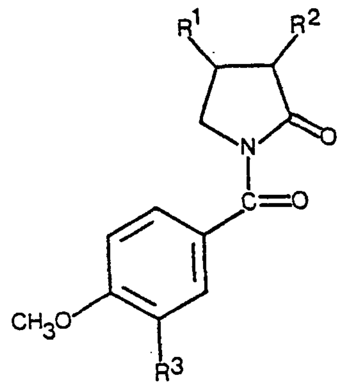

(I)

worin eines von $R^1$, $R^2$ und $R^3$ Hydroxy und die beiden anderen Wasserstoff bedeuten.

Diese Verbindungen sind neu und zeichnen sich durch wertvolle pharmakodynamische Eigenschaften aus.

Gegenstand der vorliegenden Erfindung sind Verbindungen der allgemeinen Formel I als solche und als pharmazeutische Wirkstoffe, die Herstellung dieser Verbindungen und Arzneimittel, enthaltend eine Verbindung der allgemeinen Formel I. Die Verbindungen der allgemeinen Formel I eignen sich zur Verwendung bei der Bekämpfung, bzw. Verhütung von Krankheiten bzw. bei der Verbesserung der Gesundheit, insbesondere bei der Bekämpfung bzw. Verhütung der cerebralen Insuffizienz bzw. bei der Verbesserung der intellektuellen Leistungsfähigkeit.

In der DE-A 2 413 935 und der DE-A 2 541 855 werden 4-(Polyalkoxy-phenyl)-2-pyrrolidinone mit zentral-depressiven, apomorphin-antagonistischen und antinocizeptiven Wirkungen beschrieben.

Die eingangs definierte allgemeinen Formel I umfasst drei Verbindungen, nämlich 1-(p-Methoxybenzoyl)-3-hydroxy-2-pyrrolidinon, 1-(p-Methoxybenzoyl)-4-hydroxy-2-pyrrolidinon und 1-(3-Hydroxy-4-methoxybenzoyl)-2-pyrrolidinon. Das 1-(p-Methoxybenzoyl)-3-hydroxy-2-pyrrolidinon und das 1-(p-Methoxybenzoyl)-4-hydroxy-2-pyrrolidinon enthalten je ein asymmetrisches Kohlenstoffatom ; die vorliegende Erfindung umfasst sowohl die optisch einheitlichen enantiomeren Formen dieser beider Verbindungen als auch Gemische davon (insbesondere die Racemate). Besonders bevorzugt ist das (R)-1-(p-Methoxybenzoyl)-3-hydroxy-2-pyrrolidinon ; die entsprechende (R,S)-Verbindung ist ebenfalls bevorzugt.

Die Pyrrolidin-Derivate der allgemeinen Formel I können erfindungsgemäss dadurch hergestellt werden, dass man

a) aus einem Pyrrolidin-Derivat der allgemeinen Formel

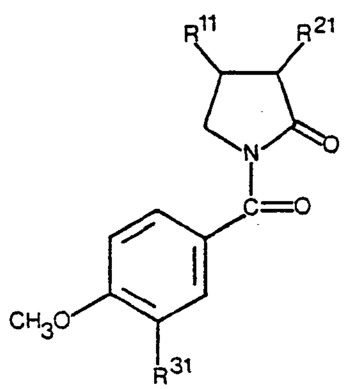

(II)

worin eines von $R^{11}$, $R^{21}$ und $R^{31}$ eine geschützte Hydroxygruppe und die beiden anderen Wasserstoff bedeuten, die Schutzgruppe entfernt oder

b) ein Pyrrolidin-Derivat der allgemeinen Formel

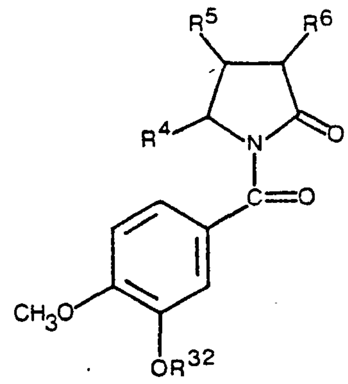

(III)

worin eines von $R^4$ und $R^6$ Wasserstoff und das andere zusammen mit $R^5$ eine zweite C-C-Bindung bedeutet und $R^{32}$ Wasserstoff oder eine reduktiv abspaltbare Schutzgruppe bedeutet, reduziert.

Nach einem ersten Verfahrensaspekt können Pyrrolidin-Derivate der allgemeinen Formel I demnach erfindungsgemäss dadurch hergestellt werden, dass man aus einem Pyrrolidin-Derivat der allgemeinen Formel II die Schutzgruppe entfernt. Als Schutzgruppen eignen sich selbstverständlich nur solche, welche durch Methoden abgespalten werden können, bei welchen diese Schutzgruppen selektiv entfernt werden, ohne dass andere im Molekül vorhandene Strukturelemente in Mitleidenschaft gezogen werden. Die Entfernung der Schutzgruppe aus den Pyrrolidin-Derivaten der allgemeinen Formel II erfolgt nach an sich bekannten Methoden, wobei natürlich für die Wahl der zur Anwendung gelangenden Methode die Natur der zu entfernenden Schutzgruppe in Betracht gezogen werden muss und zu beachten ist, dass nur die Schutzgruppe selektiv entfernt, andere im Molekül vorhandene Strukturelemente jedoch nicht in Mitleidenschaft gezogen werden sollen.

Die eingangs definierte Formel II umfasst erstens Verbindungen der allgemeinen Formel

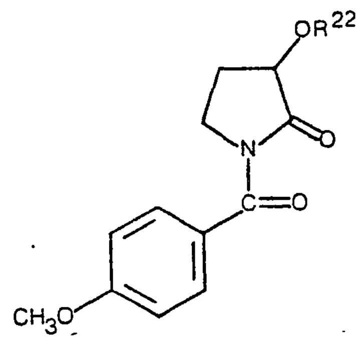

(IIa)

worin $R^{22}$ eine Schutzgruppe bedeutet, zweitens Verbindungen der Formel

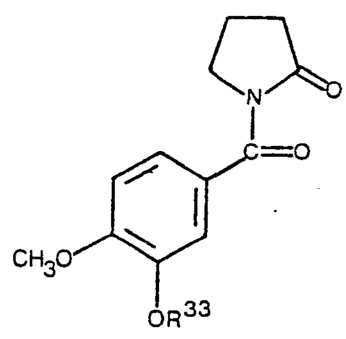

(IIb)

worin $R^{33}$ eine Schutzgruppe bedeutet, und drittens Verbindungen der Formel

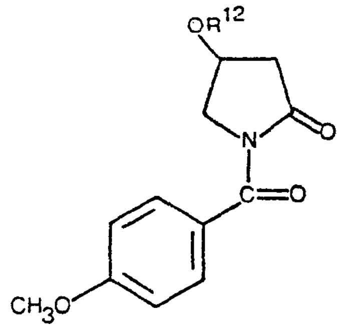

(IIc)

worin $R^{12}$ eine Schutzgruppe bedeutet.

Als Schutzgruppen in den Verbindungen der Formel IIa (durch das Symbol $R^{22}$ wiedergegeben) eignen sich beispielsweise leicht abspaltbare Alkyl- und Aralkylgruppen, wie substituierte Tritylgruppen (z. B. p-Methoxytrityl, p,p'-Dimethoxytrityl oder p,p',p'''-Trimethoxytrityl) und dergleichen ; leicht abspaltbare metallorganische Gruppen, insbesondere Trialkylsilylgruppen, wie Trimethylsilyl, und dergleichen ; leicht abspaltbare Acetal- und Ketalschutzgruppen, wie Hexahydropyran-2-yl, 4-Methoxyhexahydropyran-4-yl und dergleichen ; leicht abspaltbare Acylgruppen, wie Acetyl, Chloracetyl, Trifluoracetyl, Methoxyacetyl, Phenoxyacetyl, Benzyloxycarbonyl, Trichloräthoxycarbonyl, Tribromäthoxycarbonyl, Benzoylformyl und dergleichen, usw.

Methoden für die Entfernung der Reste, welche als Beispiele für in Betracht kommende Schutzgruppen ($R^{22}$) in Verbindungen der Formel IIa erwähnt wurden, sind in der Literatur beschrieben und demnach jedem Fachmann geläufig. Beispielsweise kann man die erwähnten Mono-, Di- und Trimethoxytritylgruppen durch Behandeln mit 80 %iger Essigsäure bei Raumtemperatur abspalten, die Trimethylsilylgruppe durch Behandeln mit verdünnter Salzsäure in Tetrahydrofuran oder dergleichen, die Hexahydropyran-2-yl-gruppe und die 4-Methoxyhexahydropyran-4-ylgruppe unter milden sauren wässrigen Bedingungen (z. B. mittels 0,1 normaler Salzsäure), die Acetylgruppe mittels Esterase-Enzymen, die Chloracetylgruppe mittels Thioharnstoff/Pyridin, die Trifluoracetylgruppe mittels Methanol, die Methoxyacetyl- und die Phenoxy-acetylgruppe mittels methanolischem Ammoniak, die Benzyloxycarbonylgruppe durch katalytische Hydrierung (z. B. über Palladium/Kohle), die Trichloräthoxycarbonyl- und die Tribromäthoxycarbonylgruppe mittels Zink-Kupfer in Eisessig bei Raumtemperatur und die Benzoylformylgruppe durch Behandeln mit wässrigem Pyridin bei Raumtemperatur.

Als Schutzgruppen in den Verbindungen der Formel IIb (durch das Symbol $R^{33}$ bezeichnet) eignen sich beispielsweise leicht abspaltbare Alkylgruppen, wie tert. Butyl und dergleichen ; leicht abspaltbare Aralkylgruppen, wie Benzyl und dergleichen ; leicht abspaltbare metallorganische Gruppen, insbesondere Trialkylsilylgruppen, wie Trimethylsilyl und dergleichen ; leicht abspaltbare Acetal- und Ketalschutzgruppen wie Hexahydropyran-2-yl und dergleichen ; leicht abspaltbare Acylgruppen wie Fluorencarbonyl, Benzyloxycarbonyl, Trichloräthoxycarbonyl, Tribromäthoxycarbonyl und dergleichen.

Methoden für die Abspaltung der Reste, welche als Beispiele für in Betracht kommende Schutzgruppen ($R^{33}$) in Verbindungen der Formel IIb erwähnt wurden, sind in der Literatur beschrieben und demnach jedem Fachmann geläufig. So lassen sich beispielsweise die Benzyl- und die Benzyloxycarbonylgruppe durch katalytische Hydrierung entfernen (z. B. über Palladium/Kohle, die tert. Butyl-, die Trimethylsilyl- und die Hexahydropyran-2-ylgruppe unter milden sauren wässrigen Bedingungen, die Fluorencarbonylgruppe mittels UV-Licht und die Trichloräthoxycarbonyl- und die Tribromäthoxycarbonylgruppe durch Erhitzen in Methanol oder mittels Zink-Kupfer in Eisessig.

Als Schutzgruppen in den Verbindungen der Formel IIc (durch das Symbol $R^{12}$ bezeichnet) eignen sich lediglich solche, bei welchen keine Elimination unter Ausbildung einer C-C-Doppelbindung im 5-gliedrigen Heterocyclus zu befürchten ist, d. h. keine Acylgruppen ; in erster Linie eignen sich Trialkylsilylgruppen, wie Trimethylsilyl, und dergleichen. Methoden für die Abspaltung derartiger Reste sind in der Literatur beschrieben und demnach jedem Fachmann geläufig ; so lässt sich beispielsweise die Trimethylsilylgruppe durch Behandlung mit verdünnter Salzsäure in Tetrahydrofuran oder dergleichen abspalten.

Nach einem zweiten Verfahrensaspekt kann man diejenige Verbindung der Formel I, worin $R^1$ und $R^2$ Wasserstoff und $R^3$ Hydroxy bedeuten, d. h. 1-(3-Hydroxy-4-methoxybenzoyl)-2-pyrrolidinon, dadurch herstellen, dass man eine Verbindung der weiter oben definierten Formel III reduziert. Die Verbindungen der Formel III enthalten im 5-gliedrigen Heterocyclus eine Doppelbindung, welche — je nach den Bedeutungen von $R^4$, $R^5$ und $R^6$ — entweder in 3,4-Stellung oder in 4,5-Stellung liegt. Es ist durchaus möglich, Gemische von 2 Verbindungen der allgemeinen Formel III einzusetzen, welche sich bezüglich der Lage dieser Doppelbindung unterscheiden. Wenn das Symbol $R^{32}$ eine reduktiv abspaltbare Schutzgruppe bedeutet, so handelt es sich beispielsweise um eine Benzylgruppe, eine Benzyloxycarbonylgruppe oder dergleichen ; selbstverständlich sollen für die Reduktion einer Verbindung der Formel III, worin $R^{32}$ eine reduktiv abspaltbare Schutzgruppe bedeutet, nur solche Methoden verwendet werden, bei welchen die Reduktion der Doppelbindung im 5-gliedrigen Heterocyclus und die Entfernung der

reduktiv abspaltbaren Schutzgruppe in einem Arbeitsgang erfolgen.

Reduktionsmethoden, welche sich für den vorliegenden Verfahrensaspekt eignen, sind in der Literatur beschrieben und demnach jedem Fachmann geläufig. Natürlich ist zu beachten, dass nur solche Methoden verwendet werden können, welche selektiv die Doppelbindung im 5-gliedrigen Heterocyclus reduzieren und eine allfällig vorhandene Schutzgruppe entfernen, ohne dass andere im Molekül vorhandene Strukturelemente in Mitleidenschaft gezogen werden. Vorzugsweise erfolgt die Reduktion der Verbindungen der allgemeinen Formel III mittels katalytisch erregtem Wasserstoff in einem unter den Reaktionsbedingungen inerten organischen Lösungsmittel, wobei als Hydrierungskatalysatoren Palladium, Platin und dergleichen in Frage kommen und als Lösungsmittel beispielsweise Essigester, Alkohole (wie Methanol, Aethanol oder dergleichen), Aether (wie Tetrahydrofuran oder dergleichen) usw.

Die Ausgangsprodukte der eingangs definierten allgemeinen Formeln II und III sind neu.

Verbindungen der Formel II, worin $R^{21}$ eine geschützte Hydroxygruppe und $R^{11}$ und $R^{31}$ Wasserstoff bedeuten, d. h. Verbindungen der allgemeinen Formel IIa, können beispielsweise dadurch hergestellt werden, dass man ein Pyrrolidin-Derivat der allgemeinen Formel

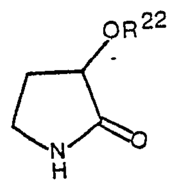

(IV)

worin $R^{22}$ obige Bedeutung besitzt, in 1-Stellung entsprechend acyliert, d.h. das Wasserstoffatom in 1-Stellung der Verbindung der Formel IV durch einen p-Methoxybenzoylrest ersetzt. Hierbei kann man Methoden verwenden, welche für derartige Acylierungen an sich allgemein bekannt sind. Als Acylierungsmittel verwendet man ein hinreichend reaktionsfähiges Derivat der p-Methoxybenzoesäure, insbesondere ein reaktionsfähiges Imidazolid oder Halogenid dieser Säure (vorzugsweise p-Methoxybenzoylchlorid).

Bei der Acylierung einer Verbindung der Formel IV mit p-Methoxybenzoylchlorid ist es zweckmässig, die Verbindung der Formel IV zunächst mit einer zur Abstraktion des Wasserstoffatoms am Stickstoffatom in 1-Stellung befähigten Base zu behandeln (z. B. mit Butyllithium) und hierauf mit p-Methoxybenzoylchlorid reagieren zu lassen. Es ist auch möglich, die Verbindung der Formel IV in Form eines reaktionsfähigen Derivates einzusetzen, in welchem am Stickstoffatom in 1-Stellung eine leicht abspaltbare Gruppe sitzt, insbesondere eine Trialkylsilylgruppe, wie 1-Trimethylsilyl ; in diesem Fall kommen natürlich als Schutzgruppen ($R^{22}$) nur Reste in Frage, welche unter den Bedingungen der Acylierung nicht in Mitleidenschaft gezogen werden.

Die Verbindungen der Formel IV können ihrerseits beispielsweise aus 3-Hydroxy-2-pyrrolidinon hergestellt werden, und zwar durch Einführung der gewünschten Schutzgruppe ; die Methoden zur Einführung der Schutzgruppen variieren je nach deren Natur, sind jedoch jedem Fachmann geläufig. Beispielsweise kann man eine Benzyloxycarbonylgruppe mittels Chlorameisensäurebenzylester und eine Chloracetylgruppe mittels Chloracetylchlorid einführen.

Gewisse Verbindungen der allgemeinen Formel IV können auch aus 4-Amino-2-hydroxybuttersäure hergestellt werden, und zwar durch Methoden, welche in einem Arbeitsgang Cyclisation und Einführung der gewünschten Schutzgruppe bewirken. So kann man beispielsweise zu 3-(Trimethylsilyloxy)-2-pyrrolidinon gelangen, indem man 4-Amino-2-hydroxybuttersäure in Gegenwart geringer Mengen von Trimethylchlorsilan mit Hexamethyldisilazan oder mit bis-(Trimethylsilyl) harnstoff oder mit bis-(Trimethylsilyl) acetamid umsetzt.

Andererseits ist es auch möglich, Verbindungen der Formel IIa aus 4-(p-Methoxybenzoylamino)-2-hydroxybuttersäure herzustellen, welche ihrerseits durch entsprechende Acylierung von 4-Amino-2-hydroxybuttersäure (beispielsweise mittels p-Methoxybenzoylchlorid) zugänglich ist. So erfolgt beispielsweise bei der Behandlung von 4-(p-Methoxybenzoylamino)-2-hydroxybuttersäure mit Essigsäureanhydrid in einem Arbeitsgang Cyclisation und Einführung der Schutzgruppe, d.h. man erhält eine Verbindung der Formel IIa, worin $R^{22}$ Acetyl bedeutet. Beispielsweise für weitere Reagenzien, mit welchen die 4-(p-Methoxybenzoylamino)-2-hydroxybuttersäure in einem Arbeitsgang in eine Verbindung der Formel IIa überführt werden kann, sind Chloressigsäureanhydrid, Methoxyessigsäureanhydrid, Trifluoressigsäureanhydrid, Hexamethyldisilazan und dergleichen ; in der erhaltenen Verbindung der Formel IIa bedeutet $R^{22}$, entsprechend dem verwendeten Reagens, Chloracetyl bzw. Methoxyacetyl bzw. Trifluoracetyl bzw. Trimethylsilyl oder dergleichen.

Weiterhin ist es auch möglich, Derivate der 4-(p-Methoxybenzoylamino)-2-hydroxybuttersäure, deren Hydroxygruppe geschützt ist, zu entsprechenden Verbindungen der Formel IIa zu cyclisieren ; zur Herstellung der erwähnten Derivate der 4-(p-Methoxybenzoylamino)-2-hydroxybuttersäure geht man von Derivaten der 4-Amino-2-hydroxybuttersäure aus, deren Hydroxygruppe bereits durch die gewünschte Schutzgruppe geschützt ist (und welche nach an sich bekannten Methoden leicht hergestellt werden können), und acyliert deren Aminogruppe mit einem hinreichend reaktiven Derivat der p-Methoxybenzoesäure, beispielsweise mit p-Methoxybenzoylchlorid.

Die Verbindungen der allgemeinen Formel IIa weisen in 3-Stellung des 5-gliedrigen Heterocyclus ein asymmetrisches Kohlenstoffatom auf. Die diesbezüglichen stereochemischen Verhältnisse bestimmen die stereochemischen Verhältnisse bei der aus den Verbindungen der Formel IIa herstellbaren entsprechenden Verbindung der Formel I, worin $R^2$ Hydroxy und $R^1$ und $R^3$ Wasserstoff bedeuten, d. h. 1-(p-Methoxybenzoyl)-3-hydroxy-2-pyrrolidinon. Die stereochemischen Verhältnisse in 3-Stellung des 5-gliedrigen Heterocyclus der Verbindungen der Formel IIa werden ihrerseits durch die bei der Herstellung der Verbindungen der Formel IIa verwendeten Vorprodukte und/oder Methoden bestimmt. Es ist für jeden Fachmann klar, wie er im Hinblick auf die soeben geschilderten Verhältnisse erfindungsgemäss zu optisch aktivem oder racemischem 1-(p-Methoxybenzoyl)-3-hydroxy-2-pyrrolidinon gelangen kann.

So kann man beispielsweise zu (R)-1-(p-methoxybenzoyl)-3-hydroxy-2-pyrrolidinon gelangen, indem man (R)-4-Amino-2-hydroxybuttersäure mittels p-Methoxybenzoylchlorid acyliert und die erhaltene (R)-4-(p-Methoxybenzoylamino)-2-hydroxybuttersäure mittels Chloressigsäureanhydrid in (R)-1-(p-Methoxybenzoyl)-2-oxo-3-pyrrolidinyl-chloracetat überführt und aus diesem die Chloracetylgruppe abspaltet oder die (R)-4-(p-Methoxybenzoylamino)-2-hydroxybuttersäure mittels Trifluoressigsäureanhydrid in (R)-1-(p-Methoxybenzoyl)-2-oxo-3-pyrrolidinyl-trifluoracetat überführt und aus diesem die Trifluoracetylgruppe abspaltet.

Verbindungen der Formel II, worin $R^{11}$ und $R^{21}$ Wasserstoff und $R^{31}$ eine geschützte Hydroxygruppe bedeuten, d. h. Verbindungen der Formel IIb, können beispielsweise dadurch hergestellt werden, dass man eine Verbindung der allgemeinen Formel

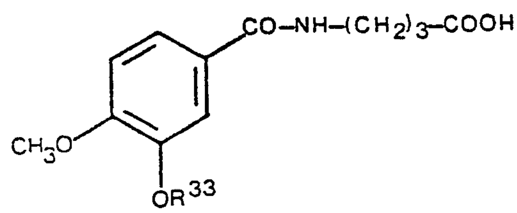

(V)

worin $R^{33}$ obige Bedeutung besitzt, cyclisiert. Bei dieser Cyclisation wird ein Mol Wasser abgespalten, und man verwendet demnach hierfür Methoden, welche für derartige dehydratisierende Cyclisationen an sich allgemein gebräuchlich sind, in erster Linie Behandlung mit einem wasserabspaltenden Mittel, wie niedere Alkancarbonsäureanhydride (z. B. Essigsäureanhydrid), substituierte Essigsäureanhydride, Thionylchlorid, Polyphosphorsäure und dergleichen und/oder Erhitzen. Verbindungen der Formel V ihrerseits erhält man beispielsweise durch Acylierung von 4-Aminobuttersäure mit einem hinreichend reaktionsfähigen Derivat (insbesondere einem reaktionsfähigen Halogenid, vorzugsweise dem Chlorid) einer Säure der allgemeinen Formel

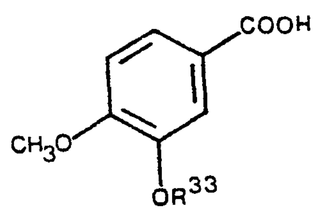

(VI)

worin $R^{33}$ obige Bedeutung besitzt.

Säuren der obigen Formel VI und reaktionsfähige Derivate davon (beispielsweise 3-Benzyloxy-4-methoxybenzoylchlorid) sind bekannt oder nach an sich bekannten Methoden leicht zugänglich.

Zu Verbindungen der Formel IIb kann man auch gelangen, indem man 2-Pyrrolidinon in 1-Stellung entsprechend acyliert, wobei man als Acylierungsmittel ein hinreichend reaktionsfähiges Derivat (insbesondere ein reaktionsfähiges Imidazolid oder Halogenid, vorzugsweise das Chlorid) einer Säure der obigen Formel VI verwendet. Bei der Acylierung von 2-Pyrrolidinon mit dem Chlorid einer Säure der obigen Formel VI arbeitet man zweckmässigerweise in Gegenwart eines inerten organischen Lösungsmittels und einer Base. Hierbei eignen sich als Lösungsmittel in — erster Linie Aether (wie Diäthyläther, Tetrahydrofuran, Dioxan usw.), aromatische Kohlenwasserstoffe (wie Toluol usw.) oder dergleichen und als Basen tert. Amine, wie Triäthylamin oder dergleichen ; die Acylierung kann auch in Pyridin durchgeführt werden, welches gleichzeitig als Lösungsmittel und als Base fungiert. Weiterhin ist es möglich, dass 2-Pyrrolidinon zunächst mit einer zur Abstraktion des Wasserstoffatoms am Stickstoffatom in 1-Stellung befähigten Base zu behandeln und hierauf mit dem Chlorid einer Säure der obigen Formel VI reagieren zu lassen. Bei dieser Ausführungsform kann als Base beispielsweise ein Alkalimetallhydrid, wie Natriumhydrid oder dergleichen, und als Lösungsmittel ein aromatischer Kohlenwasserstoff (wie Benzol), Dimethylformamid oder dergleichen verwendet werden. Es ist auch möglich, das 2-Pyrrolidinon in Form eines reaktionsfähigen Derivates einzusetzen, in welchem am Stickstoffatom in 1-Stellung eine leicht abspaltbare Gruppe sitzt, beispielsweise eine leicht abspaltbare metallorganische

Gruppe, insbesondere eine Trialkylsilylgruppe : ein bevorzugtes derartiges Derivat ist das 1-Trimethylsilyl-2-pyrrolidinon.

Weiterhin können Verbindungen der Formel IIb auch dadurch hergestellt werden, dass man eine Verbindung der allgemeinen Formel

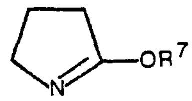

(VII)

worin $R^7$ niederes Alkyl bedeutet, mit einem Halogenid einer Säure der Formel VI behandelt, worauf das entstandene Zwischenprodukt — ohne Isolierung — hydrolysiert wird. Bei diesem Verfahren zur Herstellung von Verbindungen der Formel IIb handelt es sich um die Herstellung eines N-acylierten Lactams über einen N-acylierten Enoläther, und derartige Methoden sind an sich bekannt. Die Herstellung des N-acylierten Enoläthers erfolgt in einem geeigneten organischen Lösungsmittel (wie Benzol oder dergleichen) und gegebenenfalls in Gegenwart einer starken Base, beispielsweise in Gegenwart eines Alkalimetallhydrids, wie Lithiumhydrid. Je nach den Reaktionsbedingungen bei der Herstellung des N-acylierten Enoläthers und dessen Hydrolyse kann man neben dem gewünschten N-acylierten Lactam (d. h. im vorliegenden Fall der Verbindung der Formel IIb) in wechselnden Mengen auch den durch Ringöffnung entstehenden entsprechenden Amidoalkylester erhalten. Falls man bei der Herstellung des N-acylierten Enoläthers in einem mit Wasser nicht mischbaren Lösungsmittel arbeitet, so überwiegt bei der Hydrolyse die Bildung des gewünschten Produkts, d. h. der Verbindung der Formel IIb. Der N-acylierte Enoläther wird — wie gesagt — nicht gefasst, sondern direkt hydrolysiert, wobei die Hydrolyse in an sich bekannter Weise durch Zugabe von Wasser, von wässrigem Alkali (z. B. Lithiumhydroxidlösung) oder wässriger Säure (z. B. wässriger Salzsäure) erfolgen kann. Es ist klar, dass man bei dem soeben geschilderten, von Verbindungen der Formel VII ausgehenden Verfahren nur Verbindungen der Formel IIb herstellen kann, worin $R^{33}$ eine Schutzgruppe bedeutet, welche bei der Hydrolyse des N-acylierten Enoläthers nicht in Mitleidenschaft gezogen wird.

Verbindungen der Formel II, worin $R^{11}$ eine geschützte Hydroxygruppe und $R^{21}$ und $R^{31}$ Wasserstoff bedeuten, d. h. Verbindungen der allgemeinen Formel IIc, können beispielsweise dadurch hergestellt werden, dass man ein Pyrrolidin-Derivat der allgemeinen Formel

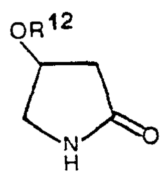

(VIII)

worin $R^{12}$ obige Bedeutung besitzt, in 1-Stellung entsprechend acyliert, d.h. das Wasserstoffatom in 1-Stellung der Verbindung der Formel VIII durch einen p-Methoxybenzoylrest ersetzt. Hierbei kann man analog vorgehen, wie bei der weiter oben beschriebenen Herstellung von Verbindungen der Formel IIa durch entsprechende Acylierung von Pyrrolidin-Derivaten der Formel IV. Die Verbindungen der Formel VIII sind bekannt oder nach an sich bekannten Methoden leicht zugänglich, insbesondere aus 4-Hydroxy-2-pyrrolidinon durch Einführung der gewünschten Schutzgruppe.

Andererseits ist es auch möglich, Verbindungen der Formel IIc aus 4-(p-Methoxybenzoylamino)-3-hydroxybuttersäure herzustellen, welche ihrerseits durch entsprechende Acylierung von 4-Amino-3-hydroxybuttersäure (beispielsweise mittels p-Methoxybenzoylchlorid) zugänglich ist. So erfolgt beispielsweise bei der Behandlung von 4-(p-Methoxybenzoylamino)-3-hydroxybuttersäure mit Hexamethyldisilazan in einem Arbeitsgang Cyclisation und Einführung der Schutzgruppe, d. h. man erhält eine Verbindung der Formel IIc, worin $R^{12}$ Trimethylsilyl bedeutet.

Die Verbindungen der allgemeinen Formel IIc weisen in 4-Stellung des 5-gliedrigen Heterocyclus ein asymmetrisches Kohlenstoffatom auf. Die diesbezüglichen stereochemischen Verhältnisse bestimmen die stereochemischen Verhältnisse bei der aus den Verbindungen der Formel IIc herstellbaren Verbindung der Formel I, worin $R^1$ Hydroxy und $R^2$ und $R^3$ Wasserstoff bedeuten, d. h. 1-(p-Methoxybenzoyl)-4-hydroxy-2-pyrrolidinon. Die stereochemischen Verhältnisse in 4-Stellung des 5-gliedrigen Heterocyclus der Verbindungen der Formel IIc werden ihrerseits durch die bei der Herstellung der Verbindungen der Formel IIc verwendeten Vorprodukte und/oder Methoden bestimmt. Es ist für jeden Fachmann klar, wie er im Hinblick auf die soeben geschilderten Verhältnisse erfindungsgemäss zu optisch aktivem oder racemischem 1-(p-Methoxybenzoyl)-4-hydroxy-2-pyrrolidinon gelangen kann.

Verbindungen der allgemeinen Formel III, worin $R^{32}$ eine reduktiv abspaltbare Schutzgruppe bedeutet, erhält man ausgehend von Verbindungen der allgemeinen Formel

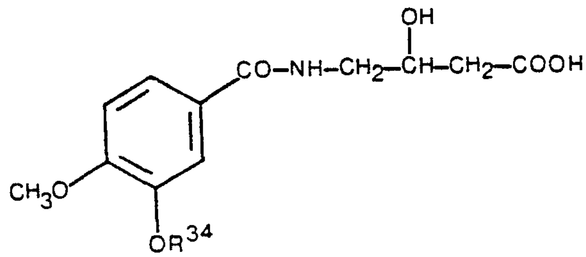

(IV)

worin $R^{34}$ eine reduktiv abspaltbare Schutzgruppe bedeutet, durch Cyclisation und Elimination, wobei in einem Arbeitsgang 2 Mol Wasser abgespalten werden. Man unterzieht demnach die Verbindung der Formel IX Methoden, welche für derartige Wasserabspaltungen allgemein gebräuchlich sind, d. h. insbesondere Behandlung mit einem geeigneten wasserabspaltenden Mittel, wie Acetanhydrid oder dergleichen.

Verbindungen der allgemeinen Formel III, worin $R^{32}$ Wasserstoff bedeutet, erhält man durch Cyclisation unter Abspaltung von 2 Mol Wasser aus Verbindungen der allgemeinen Formel

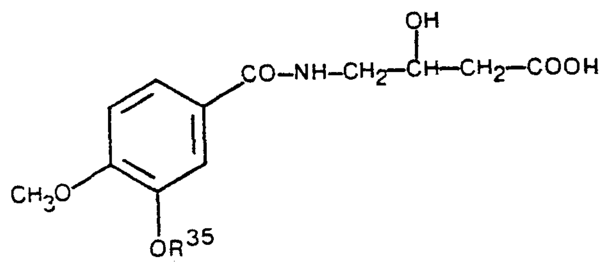

(X)

worin $R^{35}$ eine durch nicht-reduktive Methoden abspaltbare Schutzgruppe bedeutet, und anschliessende Abspaltung der erwähnten Schutzgruppe. Als Schutzgruppen für diesen Zweck (in Formel X durch das Symbol $R^{35}$ bezeichnet) eignen sich Reste wie tert. Butyl, Fluorencarbonyl, Trialkylsilyl (z. B. Trimethylsilyl) und dergleichen ; Methoden zur Abspaltung derartiger Schutzgruppen sind jedem Fachmann geläufig.

Verbindungen der Formel IX und X ihrerseits erhält man beispielsweise durch Acylierung von 4-Amino-3-hydroxy-buttersäure mit hinreichend reaktionsfähigen Derivaten von Säuren der Formel

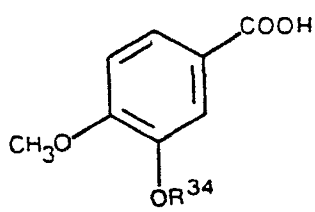

(XI)

bzw.

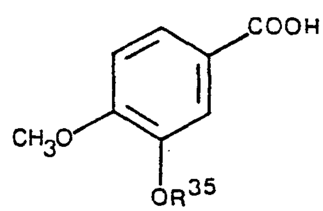

(XII)

worin $R^{34}$ und $R^{35}$ die obige Bedeutung besitzen.

Reaktionsfähige Derivate von Säuren der Formeln XI und XII sind bekannt oder nach allgemein üblichen Methoden leicht zugänglich.

Wie eingangs erwähnt, sind die Pyrrolidin-Derivate der allgemeinen Formel I neue Verbindungen mit wertvollen pharmakodynamischen Eigenschaften. Sie weisen nur eine geringe Toxizität auf, und es hat sich gezeigt, dass sie in dem nachstehend beschriebenen Tierversuch experimentell hervorgerufener cerebraler Insuffizienz entgegenzuwirken vermögen.

Posthypercapnische « Avoidance »-Acquisition

Die Test-Apparatur ist eine « shuttle box » mit einer 10 cm hohen Hürde in der Mitte und elektrifizierbarem Gitterboden. In der schalldichten Kammer ist ein Lautsprecher montiert. Eine oder drei Stunden nach Verabreichung von Kontroll- oder Präparat-Injektion werden unerfahrene Ratten (120-150 g ; 10 pro Gruppe) für 12 Sekunden in reines $CO_2$-Milieu gebracht. Eine dritte gruppe von 10 Ratten wird weder mit dem Präparat noch mit $CO_2$ behandelt. Drei Minuten nach $CO_2$-Behandlung müssen die Ratten aller drei Gruppen in der « shuttle box » in folgendem Programm einen bedingten und unbedingten Reflex erlernen : 10 Sekunden Stille — 5 Sekunden Ton (« avoidance response ») — 15 Sekunden Ton + Fuss-Schock (« escape response ») ; sechsmal hintereinander. Für jeden der sechs Einzelversuche wird die Reaktionszeit (Zeit bis die Ratte über die Hürde springt) jeder Ratte gemessen und die statistische Signifikanz der Unterschiede zwischen den verschiedenen Gruppen mittels Rang-Test berechnet.

Als « aktiv » bezeichnet man diejenige Dosis eines Präparats, welche während der sechs Einzelversuche eine signifikante Wirkung zeigt ; dabei müssen die mit dem Präparat und $CO_2$ behandelten Tiere signifikant besser lernen als die nur mit $CO_2$ behandelten Tiere und gleich gut wie die weder mit dem Präparat noch mit $CO_2$ behandelten Tiere.

In der nachfolgenden Tabelle ist angegeben, bei welchen Dosen Verbindungen der Formel I in diesem Test eine signifikante Aktivität zeigen ; die Tabelle enthält ausserdem Angaben über die Akute Toxizität der untersuchten Verbindungen (DL 50 in mg/kg bei einmaliger oraler Verabreichung an Mäuse).

| Verbindung | Signifikant wirksame Dosen | DL 50 |
|---|---|---|
| A und A' | 3 mg/kg p.o. (nach 1 Std.)<br>10 mg/kg p.o. (nach 1 Std.)<br>30 mg/kg p.o. (nach 1 Std.) | > 5 000 mg/kg p.o. |
| B | 30 mg/kg p.o. (nach 1 Std.) | 1 250-2 500 mg/kg p.o. |
| C | 10 mg/kg p.o. (nach 1 Std.) | 2 000-4 000 mg/kg p.o. |

A : (R,S)-1-(p-Methoxybenzoyl)-3-hydroxy-2-pyrrolidinon
A' : (R)-1-(p-Methoxybenzoyl)-3-hydroxy-2-pyrrolidinon
B : 1-(3-Hydroxy-4-methoxybenzoyl)-2-pyrrolidinon
C : (R,S)-1-(p-Methoxybenzoyl)-4-hydroxy-2-pyrrolidinon

Die Verbindungen der Formel I können als Heilmittel, z. B. in Form pharmazeutischer Präparate, Verwendung finden. Die pharmazeutischen Präparate können oral, z. B. in Form von Tabletten, Lacktabletten, Dragées, Hart- und Weichgelatinekapseln, Lösungen, Emulsionen oder Suspensionen, verabreicht werden. Die Verabreichung kann aber auch rektal, z. B. in Form von Suppositorien, oder parenteral, z. B. in Form von Injektionslösungen, erfolgen.

Wie eingangs erwähnt, sind Arzneimittel, enthaltend eine Verbindung der allgemeinen Formel I, ebenfalls Gegenstand der vorliegenden Erfindung ; die Herstellung derartiger Arzneimittel kann dadurch erfolgen, dass man eine oder mehrere Verbindungen der allgemeinen Formel I und erwünschtenfalls einen oder mehrere andere therapeutisch wertvolle Stoffe in eine galenische Darreichungsform bringt.

Zur Herstellung von Tabletten, Lacktabletten, Dragées und Hartgelatinekapseln können die Verbindungen der Formel I mit pharmazeutisch inerten, anorganischen oder organischen Excipientien verabreicht werden. Als solche Excipientien kann man z. B. für Tabletten, Dragées und Hartgelatinekapseln Lactose, Maisstärke oder Derivate davon, Talk, Stearinsäure oder deren Salze etc. verwenden.

Für Weichgelatinekapseln eignen sich als Excipientien z. B. vegetabile Oele, Wachse, Fette, halbfeste und flüssige Polyole etc.

Zur Herstellung von Lösungen und Sirupen eignen sich als Excipientien z. B. Wasser, Polyole, Saccharose, Invertzucker, Glukose und dergleichen.

Für Injektionslösungen eignen sich als Excipientien z. B. Wasser, Alkohole, Polyole, Glyzerin, vegetabile Oele, etc.

Für Suppositorien eignen sich als Excipientien z. B. natürliche oder gehärtete Oele, Wachse, Fette, halbflüssige oder flüssige Polyole und dergleichen.

Die pharmazeutischen Präparate können daneben noch Konservierungsmittel, Lösevermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutische wertvolle Stoffe enthalten.

Verbindungen der allgemeinen Formel I kann man bei der Bekämpfung bzw. Verhütung der cerebralen Insuffizienz bzw. bei der Verbesserung der intellektuellen Leistungsfähigkeit verwenden, beispielsweise bei cerebralen Insulten, in der Geriatrie, bei Alkoholismus usw. Die Dosierung kann innerhalb weiter Grenzen variieren und ist natürlich in jedem einzelnen Fall den individuellen Gegebenheiten anzupassen. Im Allgemeinen dürfte bei oraler Verabreichung eine Tagesdosis von etwa 10 bis 2 500 mg einer Verbindung der allgemeinen Formel I angemessen sein, wobei aber die soeben angegebene obere Grenze auch überschritten werden kann, wenn sich dies als angezeigt erweisen sollte.

In den nachfolgenden Beispielen, welche die vorliegende Erfindung illustrieren, deren Umfang jedoch in keiner Weise einschränken sollen, sind sämtliche Temperaturen in Celsiusgraden angegeben.

## Beispiel 1

a) Zu einer Suspension von 6,0 g (R,S)-4-Amino-2-hydroxy-buttersäure in 60 ml wasserfreiem o-Xylol werden 15,8 ml Hexamethyldisilazan und 0,10 ml Trimethylchlorsilan zugegeben. Die Mischung wird während 4 Stunden unter Rühren zum Sieden erhitzt und dann eingedampft, worauf der Rückstand

destilliert wird. Man erhält (R,S)-3-(Trimethylsilyloxy)-2-pyrrolidinon vom Siedepunkt 85-87°/0,02 mmHg und vom Schmelzpunkt 35-37°.

b) 5,0 g (R,S)-3-(Trimethylsilyloxy)-2-pyrrolidinon werden in 130 ml abs. Tetrahydrofuran vorgelegt, worauf bei – 30° innerhalb von 6 Minuten 14,4 ml einer etwa 2-molaren Lösung von Butyllithium in Hexan zugetropft werden. Nach 30-minütigem Rühren bei – 30° werden 4,92 g p-Methoxybenzoylchlorid, gelöst in 10 ml abs. Tetrahydrofuran, innerhalb von 5 Minuten bei – 30° zugetropft, worauf während 3 Stunden bei – 30° und während 1 Stunde bei Raumtemperatur gerührt wird. Hierauf wird eingedampft ; zum verbleibenden Rückstand, enthaltend (R,S)-1-(p-Methoxybenzoyl)-3-trimethylsilyloxy-2-pyrrolidinon, werden 30 ml Tetrahydrofuran und 6,7 ml 1N Salzsäure zugesetzt. Man rührt 7 Minuten bei Raumtemperatur, gibt dann kaltes Wasser zu und extrahiert mit Essigester. Die organische Phase wird über Natriumsulfat getrocknet und eingedampft. Der teilweise kristalline Rückstand wird in einem Gemisch von Diäthyläther und Essigester (3 : 1) verrührt ; nach Filtration und Waschen mit dem erwähnten Gemisch erhält man (R,S)-1-(p-Methoxybenzoyl)-3-hydroxy-2-pyrrolidinon vom Schmelzpunkt 124-126°.

## Beispiel 2

a) Zu 15,6 g 4-Aminobuttersäure, 12,1 g Natriumhydroxid und 160 ml ionenfreiem Wasser werden bei 26° in einer Portion 14,0 g 3-Benzyloxy-4-methoxybenzoylchlorid zugegeben und anschliessend innerhalb von 10 Minuten 30 ml Tetrahydrofuran zugetropft. Nach 2 Stunden wird das Gemisch bei einer Temperatur unterhalb von 10° mit konz. Salzsäure angesäuert. Der Niederschlag wird abgetrennt und mit Essigester verrührt, worauf man filtriert und den Filterrückstand mit Essigester wäscht. Man erhält 4-[(3-Benzyloxy-4-methoxybenzoyl) amino] buttersäure, welche nach Umkristallisieren aus Essigester bei 156-158° schmilzt.

b) 2,0 g 4-[(3-Benzyloxy-4-methoxybenzoyl) amino] buttersäure werden in 10 ml Essigsäureanhydrid 1 Stunde zum Rückfluss erhitzt. Nach Abdampfen des Essigsäureanhydrids wird der Rückstand bei Raumtemperatur mit Diäthyläther verrührt, worauf man filtriert und den Filterrückstand mit Diäthyläther wäscht. Man erhält 1-(3-Benzyloxy-4-methoxybenzoyl)-2-pyrrolidinon vom Schmelzpunkt 91-92°.

c) 2,0 g 1-(3-Benzyloxy-4 methoxybenzoyl)-2-pyrrolidinon werden in 60 ml abs. Methanol mit 0,5 g Palladiumkohle (5 %ig) unter Atmosphärendruck und bei Raumtemperatur hydriert. Der Katalysator wird abfiltriert, worauf das Filtrat eingeengt und mit Diäthyläther verrührt wird. Nach Filtration erhält man 1-(3-Hydroxy-4-methoxybenzoyl)-2-pyrrolidinon vom Schmelzpunkt 123-125°.

## Beispiel 3

21,0 g (R,S)-4-(Trimethylsilyloxy)-2-pyrrolidinon werden in 560 ml abs. Tetrahydrofuran vorgelegt, worauf bei 0° bis + 5° 60,6 ml einer 2-molaren Lösung von Butylltihium in Hexan zugetropft werden. Nach 30 Minuten werden bei 0 bis + 5° innerhalb von 10 Minuten 20,7 g p-Methoxybenzoylchlorid, gelöst in 30 ml abs. Tetrahydrofuran, zugetropft, worauf das Gemisch über Nacht im Eisschrank stehengelassen und dann eingedampft wird. Zum Rückstand, enthaltend (R,S)-1-(p-Methoxybenzoyl)-4-trimethylsilyloxy-2-pyrrolidinon, werden 140 ml abs. Tetrahydrofuran und 28 ml 1N Salzsäure zugesetzt. Dann rührt man 7 Minuten bei Raumtemperatur, gibt ionenfreies Wasser und Eis zu und extrahiert mit Essigester. Die organische Phase wird mit Wasser gewaschen, über Natriumsulfat getrocknet und eingedampft. Das zurückbleibende Oel wird über Kieselgel (Korngrösse 0,2-0,5 mm) chromatographiert. Das mit einem Gemisch von Methylenchlorid und Essigester (1 : 1) eluierte (R,S)-1-(P-Methoxybenzoyl)-4-hydroxy-2-pyrrolidinon wird aus einem Gemisch von Acetonitril-Essigester-Isopropyläther (1 : 3 : 1) kristallisiert und zeigt dann einen Schmelzpunkt von 109,5-111°.

## Beispiel 4

a) 7,25 g (R,S)-3-Hydroxy-2-pyrrolidinon werden in 140 ml Pyridin gelöst, worauf man bei 0 bis 5° 28 ml Chlorameisensäurebenzylester zugibt und danach 22 Stunden bei Raumtemperatur rührt. Das Reaktionsgemisch wird eingedampft, worauf man den Rückstand in Toluol verrührt und wiederum eindampft. Der Rückstand wird zwischen Essigsäureäthylester und Wasser verteilt. Die organische Phase wird mit Wasser gewaschen, und die Wasserphasen werden mit Essigsäureäthylester nachextrahiert. Die vereinigten Essigsäureäthylesterphasen werden über Natriumsulfat getrocknet und eingedampft. Der kristalline Rückstand wird in 30 ml Diäthyläther verrührt, und man erhält (R,S)-3-(Benzyloxycarbonyloxy)-2-pyrrolidinon vom Schmelzpunkt 81-82°.

b) 5,0 g (R,S)-3-(Benzyloxycarbonyloxy)-2-pyrrolidinon werden in Tetrahydrofuran mittels Trimethylchlorsilan und Triäthylamin silyliert. Man erhält (R,S)-3-(Benzyloxycarbonyloxy)-1-trimethylsilyl-2-pyrrolidinon vom Schmelzpunkt 56-58°.

c) 2,5 g (R,S)-3-(Benzyloxycarbonyloxy)-1-trimethylsilyl-2-pyrrolidinon werden mit 1,33 g p-Methoxybenzoylchlorid vermischt, worauf man bei Raumtemperatur rührt. Hierauf wird in einem Oelbad von 100° das entstandene Trimethylchlorsilan unter vermindertem Druck abdestilliert. Aus dem Rückstand erhält man nach Kristallisation aus Diäthyläther (R,S)-3-(Benzyloxycarbonyloxy)-1-(p-methoxybenzoyl)-2-pyrrolidinon vom Schmelzpunkt 123-124°.

d) 0,10 g (R,S)-3-(Benzyloxycarbonyloxy)-1-(p-methoxybenzoyl)-2-pyrrolidinon werden in 20 ml Tetrahydrofuran über 0,10 g 5 % Palladiumkohle mit Wasserstoff bei Atmosphärendruck hydriert. Nach Abfiltrieren des Katalysators und Einengen des Filtrats erhält man (R,S)-1-(p-Methoxy-benzoyl)-3-hydroxy-2-pyrrolidinon, welches nach Umkristallisation aus Essigsäureäthylester/n-Hexan (1 : 2) bei 124,5-126° schmilzt.

Beispiel 5

a) Zu 6,0 g (R,S)-4-Amino-2-hydroxybuttersäure und 150 ml ionenfreiem Wasser werden unter gutem Rühren 17,2 g p-Methoxybenzoylchlorid zugegeben. Danach wird mit 2N Natronlauge ein pH von 10,5 eingestellt und 70 Minuten bei Raumtemperatur weitergerührt. Die klare Lösung wird mit Eis versetzt und mit 25 %iger Salzsäure auf pH 1,4 gestellt. Der ausgefallene Festkörper wird abfiltriert und mit Wasser gewaschen. Das Filtrat wird mit Natronlauge auf pH 5,5 gebracht und im Wasserstrahlvakuum eingeengt. Die klare farblose Lösung wird mit 25 %iger Salzsäure auf pH 1,4 gestellt und zunächst mit Aether und danach mit Essigester extrahiert. Die Wasserphasen werden auf pH 5,5 gestellt, eingeengt, mit 25 %iger Salzsäure angesäuert und wiederum mit Essigester extrahiert. Der Eindampfrückstand der vereinigten Essigesterextrakte wird aus Essigester umkristallisiert. Man erhält (R,S)-4-(p-Methoxybenzoylamino)-2-hydroxybuttersäure vom Schmelzpunkt 127,5-129,5°.

b) 3,0 g (R,S)-4-(p-Methoxybenzoylamino)-2-hydroxybuttersäure werden in 20 g Chloressigsäureanhydrid 30 Minuten auf 145° erwärmt. Aus dem dunkelbraun gefärbten Reaktionsgemisch wird das Chloressigsäureanhydrid im Hochvakuum abdestilliert. Der Rückstand wird in Isopropyläther aufgekocht. Die organische Phase wird abdekantiert und mit Kohle behandelt. Die klare farblose Lösung wird im Eisbad gekühlt, und man erhält (R,S)-1-(p-Methoxybenzoyl)-2-oxo-3-pyrrolidinyl-chloracetat vom Schmelzpunkt 78-80°.

c) 1,54 g (R,S)-1-(p-Methoxybenzoyl)-2-oxo-3-pyrrolidinyl-chloracetat werden in 50 ml Pyridin unter Stickstoff mit 0,42 g Thioharnstoff und 7 ml Aethylalkohol 10 Minuten auf 100° erwärmt. Nach Abdampfen der Lösungsmittel wird der Rückstand zwischen Essigsäureäthylester und Wasser verteilt. Die organische Phase wird mehrmals mit Wasser gewaschen, und die Wasserphasen werden mit Essigsäureäthylester extrahiert. Der Eindampfrückstand der vereinigten Essigsäureäthylesterphasen wird in Isopropyläther am Rückfluss gekocht, worauf man vom unlöslichen Anteil abdekantiert. Die klare, farblose Isopropylätherphase wird im Eisbad verrührt und dann über Nacht im Eiskasten stehengelassen. Das auskristallisierte Produkt wird abfiltriert, und man erhält (R,S)-1-(p-Methoxybenzoyl)-3-hydroxy-2-pyrrolidinon vom Schmelzpunkt 125-126°.

Beispiel 6

a) 5,0 g (R,S)-3-Hydroxy-2-pyrrolidinon, 100 ml Dichlormethan und 39 ml Chloracetylchlorid werden 40 Minuten unter Rühren am Rückfluss gekocht. Danach wird das Gemisch eingedampft. Der Rückstand wird in Diäthyläther verrührt, worauf man filtriert. Der Filterkuchen wird aus Essigsäureäthylester umkristallisiert. Man erhält (R,S)-3-Chloracetoxy-2-pyrrolidinon vom Schmelzpunkt 133-134°.

b) 3,50 g (R,S)-3-Chloracetoxy-2-pyrrolidinon und 7,5 ml Trimethylchlorsilan werden in 200 ml absoluten Tetrahydrofuran gelöst, worauf man bei – 5° bis 0° 8,2 ml Triäthylamin zusetzt. Nach 4 Stunden Rühren bei 0° wird abfiltriert. Gemäss Kontrolle des Filtrates mittels NMR ist die Reaktion noch nicht beendet. Nach nochmaliger Reaktion mit Trimethylchlorsilan enthält das Filtrat 4,6 g rohes (R,S)- 2-Oxo-1-trimethylsilyl-3-pyrrolidinyl-chloracetat. Die Reinigung erfolgt mittels Vakuumdestillation ; Siedepunkt des Destillates 130°/0,02 mmHg.

c) 1,12 g (R,S)-2-Oxo-1-trimethylsilyl-3-pyrrolidinylchloracetat und 0,76 g p-Methoxybenzoylchlorid werden unter Stickstoff und unter Rühren 30 Minuten in einem Oelbad von 100° erwärmt. Gegen Ende der Reaktion wird das entstandene Trimethylchlorsilan unter vermindertem Druck abdestilliert. Das Reaktionsgemisch wird mit Diäthyläther versetzt, worauf man bei Raumtemperatur rührt und abfiltriert. Man erhält gemäss Dünnschichtchromatographie reines (R,S)-1-(p-Methoxybenzoyl)-2-oxo-3-pyrrolidinyl-chloracetat vom Schmelzpunkt 72-73°. Umkristallisation aus Isopropyläther ergibt keine Schmelzpunktserhöhung.

d) Nach den Angaben in Absatz c) von Beispiel 5 erhält man aus (R,S)-1-(p-Methoxybenzoyl)-2-oxo-3-pyrrolidinyl-chloracetat (R,S)-1-(p-Methoxybenzoyl)-3-hydroxy-2-pyrrolidinon vom Schmelzpunkt 125-126°.

Beispiel 7

5,0 g (R,S)-4-(p-Methoxybenzoylamino)-2-hydroxybuttersäure werden in 50 ml Trifluoressigsäureanhydrid und 1 g Trifluoressigsäure-natriumsalz 36 Stunden am Rückfluss gekocht. Nach Abdampfen des Lösungsmittels wird das entstandene 1-(p-Methoxybenzoyl)-2-oxo-3-pyrrolidinyl-trifluoracetat (Schmelzpunkt 107-108°) in 50 ml Methanol 30 Minuten am Rückfluss gekocht, worauf man eindampft. Der Rückstand wird zwischen Essigester und ionenfreiem Wasser verteilt. Die in Essigester löslichen Anteile werden in 60 ml eines Gemisches von Diäthyläther und Essigester (3 : 1) verrührt. Nach Filtration erhält

man rohes (R,S)-1-(p-Methoxybenzoyl)-3-hydroxy-2-pyrrolidinon vom Schmelzpunkt 121-124°. Nach Säulenchromatographie (Kieselgel, Korngrösse 0,2-0,5 mm) wird das mit Methylenchlorid und Methylenchlorid-Essigester (Gemisch 1:1) eluierte (R,S)-1-(p-Methoxybenzoyl)-3-hydroxy-2-pyrrolidinon in Diäthyläther verrührt, und man erhält ein Produkt vom Schmelzpunkt 124,5-126°.

Beispiel 8

a) 3,0 g (R,S)-4-(p-Methoxybenzoylamino)-2-hydroxybuttersäure werden in 11 ml Essigsäureanhydrid 15 Minuten zum Rückfluss erhitzt. Das Reaktionsgemisch wird im Wasserstrahlvakuum eingeengt. Man gibt sechsmal Toluol zu und dampft wieder ein. Das so erhaltene rohe Produkt wird durch Kieselgel (Körngrösse 0,2-0,5 mm) filtriert. Das mit Methylenchlorid eluierte Produkt wird im Kugelrohr destilliert, und man erhält (R,S)-1-(p-Methoxybenzoyl)-2-oxo-3-pyrrolidinylacetat vom Siedepunkt 186-188°/ 0,01 Torr.

b) 0,86 g (R,S)-1-(p-Methoxybenzoyl)-2-oxo-3-pyrrolidinylacetat werden in 43 ml 0,05 molarem Kalium-Natriumphosphatpuffer vom pH 6,9 mit 1 080 Einheiten Esteraseenzym versetzt, worauf man 5 1/2 Stunden bei Raumtemperatur rührt und danach mit Essigester extrahiert. Die Essigesterphase wird mit Wasser gewaschen. Die Wasserphasen werden mit Essigester nachextrahiert. Die vereinigten Essigesterextrakte werden über Natriumsulfat getrocknet, filtriert und eingedampft. Im Rückstand kann (R,S)-1-(p-Methoxybenzoyl)-3-hydroxy-2-pyrrolidinon nachgewiesen werden.

Beispiel 9

a) 5,0 g (R,S)-3-Acetoxy-2-pyrrolidinon und 13,2 ml Trimethylchlorsilan werden in 150 ml absolutem Tetrahydrofuran gelöst, worauf bei – 5° bis 0° unter Rühren 14,7 ml Triäthylamin zugetropft werden. Man lässt 3 Stunden bei 0° weiterrühren und filtriert danach ab. Das Filtrat wird eingedampft und der Rückstand im Vakuum destilliert. Man erhält (R,S)-2-Oxo-1-trimethylsilyl-3-pyrrolidinylacetat vom Siedepunkt 120°/0,02 mmHg.

b) 1,0 g (R,S)-2-Oxo-1-trimethylsilyl-3-pyrrolidinylacetat und 0,77 g p-Methoxybenzoylchlorid werden unter Stickstoff unter Rühren in einem Oelbad von 100° 45 Minuten erwärmt ; gegen Ende der Reaktion wird das entstandene Trimethylchlorsilan unter vermindertem Druck entfernt. Der ölige Rückstand wird durch Kieselgel (Korngrösse 0,2-0,5 mm) filtriert. Das mit Methylenchlorid eluierte (R,S)-1-(p-Methoxybenzoyl)-2-oxo-3-pyrrolidinylacetat hat einen Siedepunkt von 185-190°/0,01 mmHg.

c) Nach den Angaben in Absatz b) von Beispiel 8 erhält man aus (R,S)-1-(p-Methoxybenzoyl)-2-oxo-3-pyrrolidinylacetat (R,S)-1-(p-Methoxybenzoyl)-3-hydroxy-2-pyrrolidinon.

Beispiel 10

a) Zu 6,0 g (R)-4-Amino-2-hydroxybuttersäure und 150 ml ionenfreiem Wasser werden unter gutem Rühren 17,2 g p-Methoxybenzoylchlorid zugegeben. Danach wird mit 2N Natronlauge ein pH von 10,5 eingestellt und 70 Minuten bei Raumtemperatur weitergerührt. Die klare Lösung wird mit Eis versetzt und mit 25 %iger Salzsäure auf pH 1,4 gestellt. Der ausgefallene Festkörper wird abfiltriert und mit Wasser gewaschen. Das Filtrat wird mit Natronlauge auf pH 5,5 gebracht und im Wasserstrahlvakuum eingeengt. Die klare farblose Lösung wird mit 25 %iger Salzsäure auf pH 1,4 gestellt und zunächst mit Aether und danach mit Essigester extrahiert. Die Wasserphasen werden auf pH 5,5 gestellt, eingeengt, mit 25 %iger Salzsäure angesäuert und mit Essigester extrahiert. Der Eindampfrückstand der vereinigten Essigesterextrakte wird aus Essigester umkristallisiert. Man erhält (R)-4-(p-Methoxybenzoylamino)-2-hydroxybuttersäure vom Schmelzpunkt 105-106° ; $[\alpha]_D^{20}$ : – 12,4°, $[\alpha]_{365}^{20}$ : – 60,5° (Wasser, c = 1,0). Aus den Mutterlaugen kann eine weitere Portion (R)-4-(p-Methoxybenzoylamino)-2-hydroxybuttersäure vom Schmelzpunkt 103,5-104,5° isoliert werden.

b) Nach den Angaben in Absatz b) von Beispiel 5 erhält man aus der (R)-4-(p-Methoxybenzoylamino)-2-hydroxybuttersäure (R)-1-(p-Methoxybenzoyl)-2-oxo-3-pyrrolidinyl-chloracetat ; $[\alpha]_D^{20}$ : + 116°, $[\alpha]_{546}^{20}$ : + 144°, $[\alpha]_{365}^{20}$ : + 717° (Chloroform, c = 1).

c) 5,0 g (R)-1-(p-Methoxybenzoyl)-2-oxo-3-pyrrolidinylchloracetat werden entsprechend den Angaben in Absatz c) von Beispiel 5 in Pyridin mit Thioharnstoff und Aethylalkohol behandelt ; die Reaktionszeit beträgt 45 Minuten. Der beim Aufarbeiten erhaltene Rückstand der Essigsäureäthylesterextrakte wird in Aether verrührt, worauf die unlöslichen Anteile abfiltriert und aus Isopropyläther umkristallisiert werden. Man erhält (R)-1-(p-Methoxybenzoyl)-3-hydroxy-2-pyrrolidinon vom Schmelzpunkt 123-124° ; $[\alpha]_D^{20}$ : + 214°, $[\alpha]_{546}^{20}$ : + 264°, $[\alpha]_{365}^{20}$ : + 1 212° (Chloroform, c = 1,0).

Beispiel 11

1,05 g (R)-4-(p-Methoxybenzoylamino)-2-hydroxybuttersäure werden in 8,5 ml Trifluoressigsäureanhydrid und 0,2 g Trifluoressigsäure-Natriumsalz 48 Stunden unter Rühren am Rückfluss gekocht. Nach Eindampfen des Reaktionsgemisches wird der Rückstand zweimal mit Toluol geschüttelt, und das Toluol

wird danach im Vakuum abgedampft. Der Rückstand, enthaltend (R)-1-(p-Methoxybenzoyl)-2-oxo-3-pyrrolidinyl-trifluoracetat, wird in absolutem Methanol 30 Minuten am Rückfluss gekocht. Nach Abdampfen des Methanols wird der Rückstand in 400 ml Isopropyläther aufgekocht, worauf man abdekantiert, auf 140 ml einengt und danach bei Raumtemperatur rührt. Man filtriert den Festkörper ab und erhält (R)-1-(p-Methoxybenzoyl)-3-hydroxy-2-pyrrolidinon vom Schmelzpunkt 122,5-123° ; $[\alpha]_D^{20}$ : + 207°, $[\alpha]_{546}^{20}$ : + 256°, $[\alpha]_{365}^{20}$ : + 1 172° (Chloroform, c = 1,0).

Aus dem Filtrat und den in Dichlormethan löslichen Anteilen der in Isopropyläther unlöslichen Anteile kann durch Verrühren in Diäthyläther bei Raumtemperatur weiteres (R)-1-(p-Methoxybenzoyl)-3-hydroxy-2-pyrrolidinon vom Schmelzpunkt 121-122,5° isoliert werden ; $[\alpha]_D^{20}$ : + 200,3°, $[\alpha]_{546}^{20}$ : + 247,5°, $[\alpha]_{365}^{20}$ : + 1 133,7° (Chloroform, c = 1,0).

## Beispiel 12

a) 18,1 g 4-Amino-3-hydroxybuttersäure werden in 176 ml 2N Natronlauge gelöst. Hierzu werden innerhalb von 2 Minuten 14,0 g 3-Benzyloxy-4-methoxybenzoylchlorid und danach 30 ml Tetrahydrofuran zugegeben. Bei Raumtemperatur wird 2 Stunden intensiv nachgerührt, danach mit Eis versetzt und mit konz. Salzsäure angesäuert. Der Ausgefallene Festkörper wird abfiltriert und mit Wasser gewaschen ; nach dem Trocknen wird der Filterkuchen in Essigsäureäthylester aufgekocht, worauf man die unlöslichen Anteile aus Tetrahydrofuran-n-Hexan (3,6 : 1) umkristallisiert. Man erhält 4-[(3-Benzyloxy-4-methoxybenzoyl) amino]-3-hydroxybuttersäure vom Schmelzpunkt 158-160°.

b) 5,0 g 4-[(3-Benzyloxy-4-methoxybenzoyl) amino]-3-hydroxybuttersäure werden in 15 ml Essigsäureanhydrid 20 Minuten am Rückfluss gekocht. Nach Abdampfen des Essigsäureanhydrids im Vakuum wird der Rückstand an 20 g Kieselgel (Korngrösse 0,2 bis 0,5 mm) chromatographiert. Das mit Toluol und Dichlormethan eluierte Produktgemisch wird mittels Druckchromatographie an Kieselgel (Korngrösse 0,063-0,2 mm) aufgetrennt. Das mit Essigester-n-Hexan (9 : 1) eluierte fast reine 1-(3-Benzyloxy-4-methoxybenzoyl)-pyrrolin-2-on zeigt nach Verrühren in Diäthyläther einen Schmelzpunkt von 113-114°. Das Dünnschichtchromatogramm zeigt nur einen Fleck ; das NMR-Spektrum stimmt mit der angenommenen Struktur überein.

c) 150 mg 1-(3-Benzyloxy-4-methoxybenzoyl) pyrrolin-2-on werden in 100 ml Essigsäureäthylester gelöst und über 150 mg 5 %iger Palladiumkohle mit Wasserstoff bei Atmosphärendruck hydriert. Nach Abfiltrieren des Katalysators und Einengen wird der Rückstand in Diäthyläther bei Raumtemperatur verrührt. Nach Abfiltrieren erhält man 1-(3-Hydroxy-4-methoxybenzoyl)-2-pyrrolidinon vom Schmelzpunkt 122-124°.

## Beispiel 13

a) Durch Erwärmen von äquimolaren Mengen von 3-Benzyloxy-4-methoxybenzoylchlorid und 1-Trimethylsilyl-2-pyrrolidinon in einem Oelbad von 100° in Analogie zu den Angaben in Absatz b) von Beispiel 9 erhält man 1-(3-Benzyloxy-4-methoxybenzoyl)-2-pyrrolidinon. Nach Verrühren in Diäthyläther und Umkristallisation aus Essigsäureäthylester-n-Hexan (3 : 1) zeigt die Substanz einen Schmelzpunkt von 90,5-91°.

b) Nach den Angaben in Absatz b) von Beispiel 2 erhält man aus 1-(3-Benzyloxy-4-methoxybenzoyl)-2-pyrrolidinon 1-(3-Hydroxy-4-methoxybenzoyl)-2-pyrrolidinon vom Schmelzpunkt 123-125°.

## Beispiel 14

a) 10,7 g (R,S)-4-Amino-3-hydroxybuttersäure und 7,2 g Natriumhydroxid werden in 90 ml ionenfreiem Wasser gelöst. Bei 26° werden innerhalb von 2 Minuten unter Rühren 5,1 g p-Methoxybenzoylchlorid zugegeben, wobei die Temperatur auf 31° ansteigt. Das Gemisch wird bei Raumtemperatur 1 Stunde intensiv nachgerührt und danach bei 5 bis 10° Innentemperatur mit konz. Salzsäure angesäuert. Der ausgefallene Festkörper wird abfiltriert und mit ionenfreiem Wasser bis zur schwach sauren Reaktion (pH etwa 4) gewaschen. Man erhält (R,S)-4-(p-Methoxybenzoylamino)-3-hydroxybuttersäure vom Schmelzpunkt 119-121°. Aus dem Filtrat kann nach Einengen eine weitere Portion desselben Produktes von gleichem Schmelzpunkt abfiltriert werden.

b) 2,0 g (R,S)-4-(p-Methoxybenzoylamino)-3-hydroxybuttersäure werden in 30 ml o-Xylol mit 12 ml Hexamethyldisilazan und 0,10 ml Trimethylchlorsilan 52 Stunden unter Rühren am Rückfluss gekocht. Das Reaktionsgemisch wird eingedampft. Zum verbleibenden Rückstand gibt man viermal Toluol zu und dampft ein. Der Rückstand, enthaltend (R,S)-1-(p-Methoxybenzoyl)-5-oxo-3-trimethylsilyloxypyrrolidin, wird in 20 ml Essigsäureäthylester und 15 ml 0,4 N Salzsäure 2 Stunden bei Raumtemperatur gerührt. Die organische Phase wird mit Wasser gewaschen. Die Wasserphasen werden mit Essigsäureäthylester nachextrahiert. Die vereinigten organischen Phasen werden eingeengt. Der verbleibende Rückstand wird durch Kieselgel (Korngrösse 0,2-0,5 mm) chromatographiert. Das mit Essigsäureäthylester eluierte Produkt wird erneut durch Kieselgel chromatographiert. Das mit Methylenchlorid/Essigsäureäthylester (1 : 1) eluierte rohe (R,S)-1-(p-Methoxybenzoyl)-4-hydroxy-2-pyrrolidinon wird bei Raumtemperatur in Diäthyläther verrührt. Man erhält ein Produkt vom Schmelzpunkt 117-119°.

Beispiel A

1-(p-Methoxybenzoyl)-3-hydroxy-2-pyrrolidinon wird als Wirkstoff zur Herstellung von Tabletten folgender Zusammensetzung verwendet :

| | pro Tablette |
|---|---|
| Wirkstoff (feingemahlen) | 25 mg |
| Milchzucker (pulverisiert) | 180 mg |
| Maisstärke (weiss) | 275 mg |
| Polyvinylpyrrolidon | 15 mg |
| Magnesiumstearat | 5 mg |
| | 500 mg |

Der feingemahlene Wirkstoff, der pulverisierte Milchzucker und ein Teil der weissen Maisstärke werden miteinander vermischt. Die Mischung wird gesiebt, worauf man sie mit einer Lösung von Polyvinylpyrrolidon in Wasser befeuchtet, knetet, feuchtgranuliert und trocknet. Das Granulat, der Rest der Maisstärke und das Magnesiumstearat werden gesiebt und miteinander vermischt. Das Gemisch wird zu Tabletten geeigneter Form und Grösse verpresst.

Beispiel B

1-(p-Methoxybenzoyl)-3-hydroxy-2-pyrrolidinon wird als Wirkstoff zur Herstellung von Tabletten folgender Zusammensetzung verwendet :

| | pro Tablette |
|---|---|
| Wirkstoff (feingemahlen) | 20 mg |
| Maisstärke (weiss) | 220 mg |
| Milchzucker | 70 mg |
| Mikrokristalline Cellulose | 40 mg |
| Polyvinylpyrrolidon | 20 mg |
| Natrium-carboxymethylstärke | 23 mg |
| Magnesiumstearat | 2 mg |
| | 395 mg |

Der feingemahlene Wirkstoff, ein Teil der weissen Maisstärke, der Milchzucker, die mikrokristalline Cellulose und das Polyvinylpyrrolidon werden miteinander vermischt. Die Mischung wird gesiebt und mit dem Rest der weissen Maisstärke und Wasser zu einem Granulat verarbeitet, welches getrocknet und gesiebt wird. Dann gibt man die Natrium-carboxymethylstärke und das Magnesiumstearat zu, vermischt und verpresst das Gemisch zu Tabletten geeigneter Grösse, welche eine Kreuzbruchrille aufweisen.

Beispiel C

1-(p-Methoxybenzoyl)-3-hydroxy-2-pyrrolidinon wird als Wirkstoff zur Herstellung von Tabletten folgender Zusammensetzung verwendet :

| | pro Tablette |
|---|---|
| Wirkstoff (feingemahlen) | 125 mg |
| Maisstärke (weiss) | 560 mg |
| Milchzucker | 165 mg |
| Mikrokristalline Cellulose | 70 mg |
| Polyvinylpyrrolidon | 35 mg |
| Natrium-carboxymethylstärke | 40 mg |
| Magnesiumstearat | 5 mg |
| | 1 000 mg |

Der feingemahlene Wirkstoff, ein Teil der weissen Maisstärke, der Milchzucker, die mikrokristalline Cellulose und das Polyvinylpyrrolidon werden miteinander vermischt. Die Mischung wird gesiebt und mit dem Rest der weissen Maisstärke und Wasser zu einem Granulat verarbeitet, welches getrocknet und gesiebt wird. Dann gibt man die Natrium-carboxymethylstärke und das Magnesiumstearat zu, vermischt und verpresst das Gemisch zu Tabletten geeigneter Grösse, welche eine Kreuzbruchrille aufweisen.

Beispiel D

1-(p-Methoxybenzoyl)-3-hydroxy-2-pyrrolidinon wird als Wirkstoff zur Herstellung von Doppelampullen folgender Zusammensetzung verwendet :

Wirkstofflösung

| | | |
|---|---|---|
| Wirkstoff | | 25 mg |
| Polyäthylenglykol | ad | 5 ml |

Diluens

| | |
|---|---|
| Wasser zu Injektionszwecken | 5 ml |

Vor der Injektion ist das Diluens dem Inhalt der Wirkstoffampulle zuzufügen. Man erhält 10 ml einer spritzfertigen Lösung, enthaltend 25 mg Wirkstoff.

Beispiel E

1-(p-Methoxybenzoyl)-3-hydroxy-2-pyrrolidinon wird als Wirkstoff zur Herstellung von Doppelampullen folgender Zusammensetzung verwendet :

Wirkstofflösung

| | | |
|---|---|---|
| Wirkstoff | | 25 mg |
| Glycofurol | ad | 3,5 ml |

Diluens

| | | |
|---|---|---|
| Natriumchlorid | | 67,5 mg |
| Wasser zu Injektionszwecken | ad | 6,5 ml |

Vor der Injektion ist das Diluens dem Inhalt der Wirkstoffampulle zuzufügen. Man erhält 10 ml einer spritzfertigen Lösung, enthaltend 25 mg Wirkstoff.

Beispiel F

1-(p-Methoxybenzoyl)-3-hydroxy-2-pyrrolidinon wird als Wirkstoff zur Herstellung von Doppelampullen folgender Zusammensetzung verwendet :

Wirkstofflösung

| | | |
|---|---|---|
| Wirkstoff | | 25 mg |
| Polyäthylenglykol | | 1,5 ml |
| Glycofurol | ad | 4 ml |

Diluens

| | |
|---|---|
| Wasser zu Injektionszwecken | 6 ml |

Vor der Injektion ist das Diluens dem Inhalt der Wirkstoffampulle zuzufügen. Man erhält 10 ml einer spritzfertigen Lösung, enthaltend 25 mg Wirkstoff.

**Ansprüche** (für die Vertragsstaaten : BE, CH, DE, FR, IT, LI, LU, NL, SE)

1. Pyrrolidin-Derivate der allgemeinen Formel

(Siehe die Formel Seite 16)

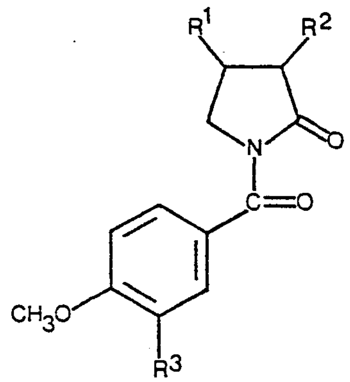

(I)

worin eines von $R^1$, $R^2$ und $R^3$ Hydroxy und die beiden anderen Wasserstoff bedeuten.

2. (R)-1-(p-Methoxybenzoyl)-3-hydroxy-2-pyrrolidinon.
3. (R, S)-1-(p-Methoxybenzoyl)-3-hydroxy-2-pyrrolidinon.
4. (R, S)-1-(p-Methoxybenzoyl)-4-hydroxy-2-pyrrolidinon.
5. 1-(3-Hydroxy-4-methoxybenzoyl)-2-pyrrolidinon.
6. Verbindungen gemäss einem der Ansprüche 1 und 3 bis 5 zur Verwendung als pharmazeutische Wirkstoffe.
7. Verbindungen gemäss einem der Ansprüche 1 und 3 bis 5 zur Verwendung bei der Bekämpfung bzw. Verhütung cerebraler Insuffizienz bzw. bei der Verbesserung der intellektuellen Leistungsfähigkeit.
8. (R)-1-(p-Methoxybenzoyl)-3-hydroxy-2-pyrrolidinon zur Verwendung als pharmazeutischer Wirkstoff.
9. (R)-1-(p-Methoxybenzoyl)-3-hydroxy-2-pyrrolidinon zur Verwendung bei der Bekämpfung bzw. Verhütung cerebraler Insuffizienz bzw. bei der Verbesserung der intellektuellen Leistungsfähigkeit.
10. Verfahren zur Herstellung von Verbindungen gemäss einem der Ansprüche 1 bis 5, dadurch gekennzeichnet, dass man

a) aus einem Pyrrolidin-Derivat der allgemeinen Formel

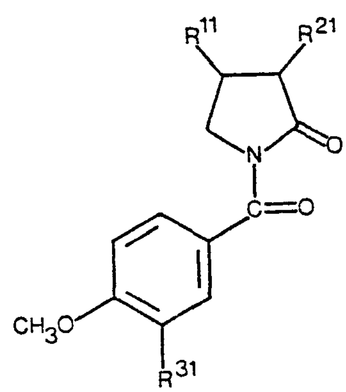

(II)

worin eines von $R^{11}$, $R^{21}$ und $R^{31}$ eine geschützte Hydroxygruppe und die beiden anderen Wasserstoff bedeuten, wobei aber für den Fall, dass $R^{11}$ eine geschützte Hydroxygruppe bedeutet, die Schutzgruppe keine Acylgruppe ist, die Schutzgruppe entfernt oder

b) ein Pyrrolidin-Derivat der allgemeinen Formel

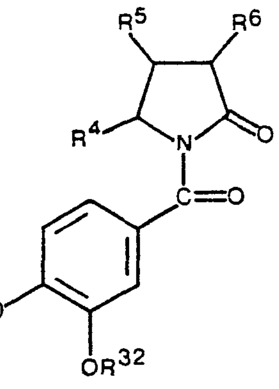

(III)

worin eines von $R^4$ und $R^6$ Wasserstoff und das andere zusammen mit $R^5$ eine zweite C—C-Bindung bedeutet und $R^{32}$ Wasserstoff oder eine reduktiv abspaltbare Schutzgruppe bedeutet, reduziert.

11. Verfahren nach Anspruch 10, dadurch gekennzeichnet, dass man (R)-1-(p-Methoxybenzoyl)-3-hydroxy-2-pyrrolidinon herstellt.

12. Arzneimittel, enthaltend eine Verbindung gemäss einem der Ansprüche 1 und 3 bis 5.

13. Der cerebralen Insuffizienz entgegenwirkendes bzw. die intellektuelle Leistungsfähigkeit verbesserndes Mittel, enthaltend eine Verbindung gemäss einem der Ansprüche 1 und 3 bis 5.

14. Arzneimittel, enthaltend (R)-1-(p-Methoxybenzoyl)-3-hydroxy-2-pyrrolidinon.

15. Der cerebralen Insuffizienz entgegenwirkendes bzw. die intellektuelle Leistungsfähigkeit verbesserndes Mittel, enthaltend (R)-1-(p-Methoxybenzoyl)-3-hydroxy-2-pyrrolidinon.

**Ansprüche** (für den Vertragsstaat AT)

1. Verfahren zur Herstellung von Pyrrolidin-Derivaten der allgemeinen Formel

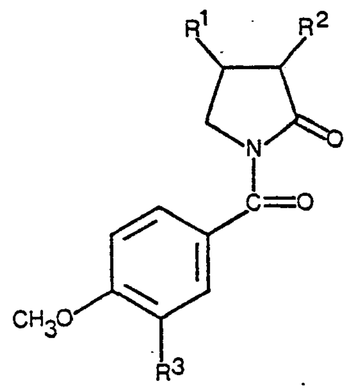

(I)

worin eines von $R^1$, $R^2$ und $R^3$ Hydroxy und die beiden anderen Wasserstoff bedeuten, dadurch gekennzeichnet, dass man

a) aus einem Pyrrolidin-Derivat der allgemeinen Formel

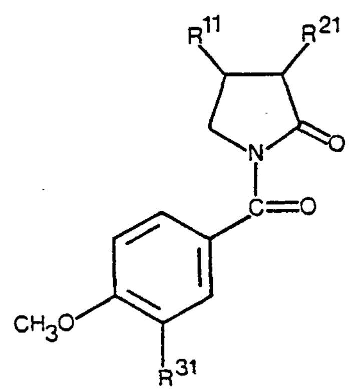

(II)

worin eines von $R^{11}$, $R^{21}$ und $R^{31}$ eine geschützte Hydroxygruppe und die beiden anderen Wasserstoff bedeuten, wobei aber für den Fall, dass $R^{11}$ eine geschützte Hydroxygruppe bedeutet, die Schutzgruppe keine Acylgruppe ist, die Schutzgruppe entfernt oder

b) ein Pyrrolidin-Derivat der allgemeinen Formel

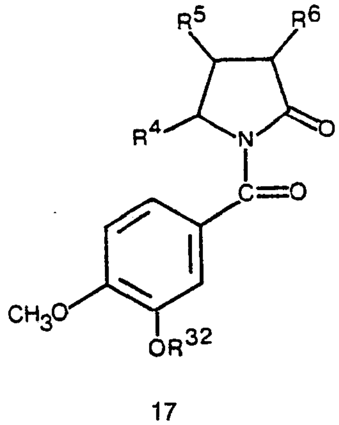

(III)

worin eines von $R^4$ und $R^6$ Wasserstoff und das andere zusammen mit $R^5$ eine zweite C—C-Bindung bedeutet und $R^{32}$ Wasserstoff oder eine reduktiv abspaltbare Schutzgruppe bedeutet, reduziert.

2. Verfahren nach Anspruch 1, dadurch gekennzeichnet, dass man (R)-1-(p-Methoxybenzoyl)-3-hydroxy-2-pyrrolidinon herstellt.

3. Verfahren nach Anspruch 1, dadurch gekennzeichnet, dass man (R,S)-1-(p-Methoxybenzoyl)-3-hydroxy-2-pyrrolidinon herstellt.

4. Verfahren nach Anspruch 1, dadurch gekennzeichnet, dass man (R,S)-1-(p-Methoxybenzoyl)-4-hydroxy-2-pyrrolidinon herstellt.

5. Verfahren nach Anspruch 1, dadurch gekennzeichnet, dass man 1-(3-Hydroxy-4-methoxybenzoyl)-2-pyrrolidinon herstellt.

**Claims** (for the Contracting States : BE, CH, DE, FR, IT, LI, LU, NL, SE)

1. Pyrrolidine derivatives of the general formula

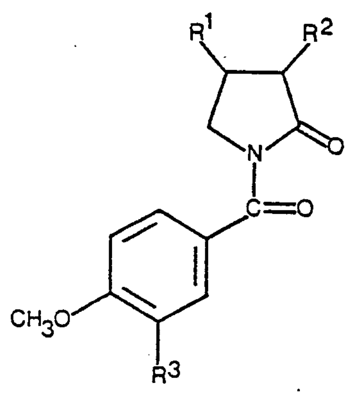

(I)

wherein one of $R^1$, $R^2$ and $R^3$ signifies hydroxy and the other two signify hydrogen.

2. (R)-1-(p-Methoxybenzoyl)-3-hydroxy-2-pyrrolidinone.

3. (R,S)-1-(p-Methoxybenzoyl)-3-hydroxy-2-pyrrolidinone.

4. (R,S)-1-(p-Methoxybenzoyl)-4-hydroxy-2-pyrrolidinone.

5. 1-(3-Hydroxy-4-methoxybenzoyl)-2-pyrrolidinone.

6. Compounds in accordance with any one of claims 1 and 3 to 5 for use as pharmaceutically active substances.

7. Compounds in accordance with any one of claims 1 and 3 to 5 for use in the control or prevention of cerebral insufficiency or in the improvement of intellectual capacity.

8. (R)-1-(p-Methoxybenzoyl)-3-hydroxy-2-pyrrolidinone for use as a pharmaceutically active substance.

9. (R)-1-(p-Methoxybenzoyl)-3-hydroxy-2-pyrrolidinone for use in the control or prevention of cerebral insufficiency or in the improvement of intellectual capacity.

10. Process for the manufacture of compounds in accordance with any one of claims 1 to 5, characterized by

a) removing the protecting group from a pyrrolidine derivative of the general formula

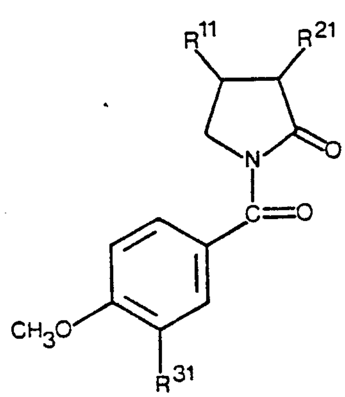

(II)

wherein one of $R^{11}$, $R^{21}$ and $R^{31}$ signifies a protected hydroxy group and the other two signify hydrogen, whereby, however, where $R^{11}$ signifies a protected hydroxy group, the protecting group is not an acyl group, or

b) reducing a pyrrolidine derivative of the general formula

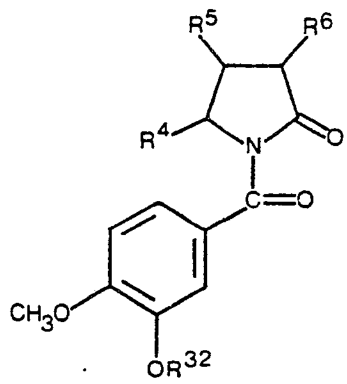

(III)

wherein one of $R^4$ and $R^6$ signifies hydrogen and the other together with $R^5$ signifies a second C—C bond and $R^{32}$ signifies hydrogen or a reductively cleavable protecting group.

11. Process according to claim 10, characterized in that (R)-1-(p-methoxybenzoyl)-3-hydroxy-2-pyrrolidinone is manufactured.

12. Medicament, containing a compound in accordance with any one of claims 1 and 3 to 5.

13. Agent counteracting cerebral insufficiency or improving intellectual capacity, containing a compound in accordance with any one of claims 1 and 3 to 5.

14. Medicament, containing (R)-1-(p-methoxybenzoyl)-3-hydroxy-2-pyrrolidinone.

15. Agent counteracting cerebral insufficiency or improving intellectual capacity, containing (R)-1-(p-methoxybenzoyl)-3-hydroxy-2-pyrrolidinone.

**Claims** (for the Contracting State AT)

1. Process for the manufacture of pyrrolidine derivatives of the general formula

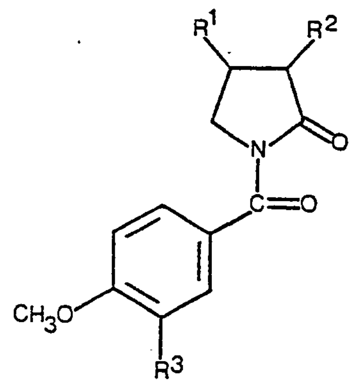

(I)

wherein one of $R^1$, $R^2$ and $R^3$ signifies hydroxy and the other two signify hydrogen, characterized by

a) removing the protecting group from a pyrrolidine derivative of the general formula

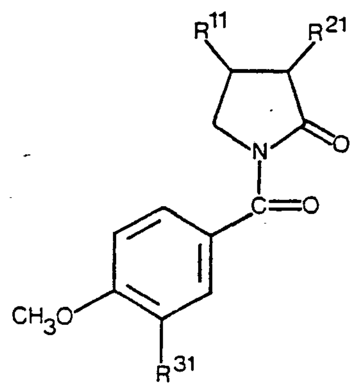

(II)

wherein one of $R^{11}$, $R^{21}$ and $R^{31}$ signifies a protected hydroxy group and the other two signify hydrogen,

whereby, however, where $R^{11}$ signifies a protected hydroxy group, the protecting group is not an acyl group, or

b) reducing a pyrrolidine derivative of the general formula

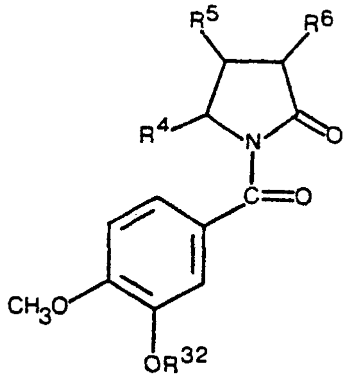

(III)

wherein one of $R^4$ and $R^6$ signifies hydrogen and the other together with $R^5$ signifies a second C—C bond and $R^{32}$ signifies hydrogen or a reductively cleavable protecting group.

2. Process according to claim 1, characterized in that (R)-1-(p-methoxybenzoyl)-3-hydroxy-2-pyrrolidinone is manufactured.

3. Process according to claim 1, characterized in that (R,S)-1-(p-methoxybenzoyl)-3-hydroxy-2-pyrrolidinone is manufactured.

4. Process according to claim 1, characterized in that (R,S)-1-(p-methoxybenzoyl)-4-hydroxy-2-pyrrolidinone is manufactured.

5. Process according to claim 1, characterized in that 1-(3-hydroxy-4-methoxybenzoyl)-2-pyrrolidinone is manufactured.

**Revendications** (pour les Etats contractants : BE, CH, DE, FR, IT, LI, LU, NL, SE)

1. Dérivés de pyrrolidine de formule générale

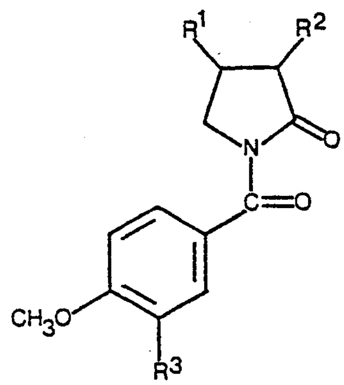

(I)

où l'un des radicaux $R^1$, $R^2$ et $R^3$ représente un hydroxy et les deux autres un hydrogène.

2. (R)-1-(p-méthoxybenzoyl)-3-hydroxy-2-pyrrolidinone.

3. (R,S)-1-(p-méthoxybenzoyl)-3-hydroxy-2-pyrrolidinone.

4. (R,S)-1-(p-méthoxybenzoyl)-4-hydroxy-2-pyrrolidinone.

5. 1-(3-hydroxy-4-méthoxybenzoyl)-2-pyrrolidinone.

6. Composés selon l'une des revendications 1 et 3 à 5 aux fins d'application comme substances actives pharmaceutiques.

7. Composés selon l'une des revendications 1 et 3 à 5 aux fins d'application pour combattre ou prévenir l'insuffisance cérébrale ou améliorer la capacité intellectuelle.

8. (R)-1-(p-méthoxybenzoyl)-3-hydroxy-2-pyrrolidinone aux fins d'application comme substance active pharmaceutique.

9. (R)-1-(p-méthoxybenzoyl)-3-hydroxy-2-pyrrolidinone aux fins d'application pour combattre ou prévenir l'insuffisance cérébrale ou améliorer la capacité intellectuelle.

10. Procédé de préparation de composés selon l'une des revendications 1 à 5, caractérisé en ce que :

a) on élimine le groupe protecteur d'un dérivé de pyrrolidine de formule générale

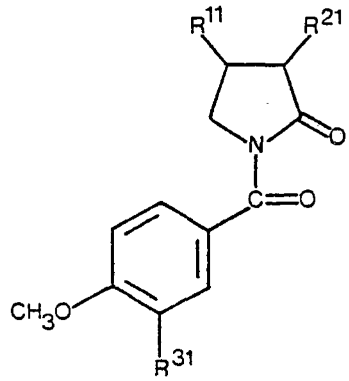

(II)

où l'un des radicaux $R^{11}$, $R^{21}$ et $R^{31}$ représente un groupe hydroxy protégé et les deux autres un hydrogène, mais où au cas où $R^{11}$ représente un groupe hydroxy protégé, le groupe protecteur n'est pas un groupe acyle ; ou

b) on réduit un dérivé de pyrrolidine de formule générale

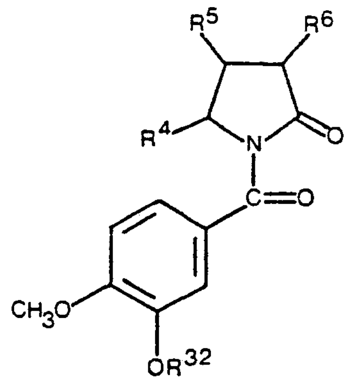

(III)

où l'un des radicaux $R^4$ et $R^6$ représente un hydrogène et l'autre forme avec $R^5$ une deuxième liaison C—C et $R^{32}$ représente un hydrogène ou un groupe protecteur séparable par réduction.

11. Procédé selon la revendication 10, caractérisé en ce qu'on prépare la (R)-1-(p-méthoxybenzoyl)-3-hydroxy-2-pyrrolidinone.

12. Médicament contenant un composé selon l'une des revendications 1 et 3 à 5.

13. Agent luttant contre l'insuffisance cérébrale ou améliorant la capacité intellectuelle, contenant un composé selon l'une des revendications 1 et 3 à 5.

14. Médicament contenant la (R)-1-(p-méthoxybenzoyl)-3-hydroxy-2-pyrrolidinone.

15. Agent luttant contre l'insuffisance cérébrale ou améliorant la capacité intellectuelle, contenant la (R)-1-(p-méthoxybenzoyl)-3-hydroxy-2-pyrrolidinone.

**Revendications** (pour l'Etat contractant AT)

1. Procédé de préparation de dérivés de pyrrolidine de formule générale

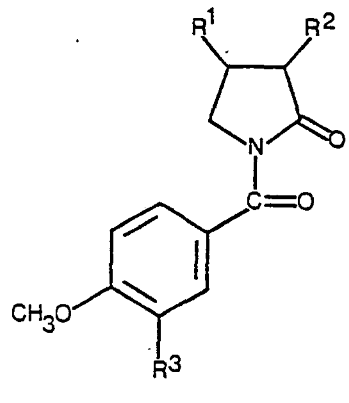

(I)

où l'un des radicaux $R^1$, $R^2$ et $R^3$ représente un hydroxy et les deux autres un hydrogène, caractérisé en ce que :

a) on élimine le groupe protecteur d'un dérivé de pyrrolidine de formule générale

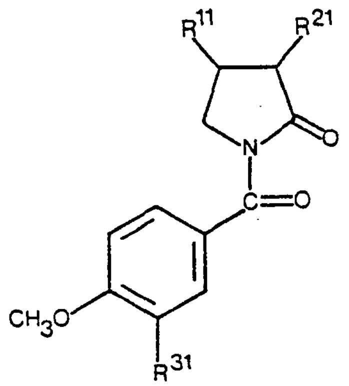

(II)

où l'un des radicaux $R^{11}$, $R^{21}$ et $R^{31}$ représente un groupe hydroxy protégé et les deux autres un hydrogène, mais où au cas où $R^{11}$ représente un groupe hydroxy protégé, le groupe protecteur n'est pas un groupe acyle, ou

b) on réduit un dérivé de pyrrolidine de formule générale

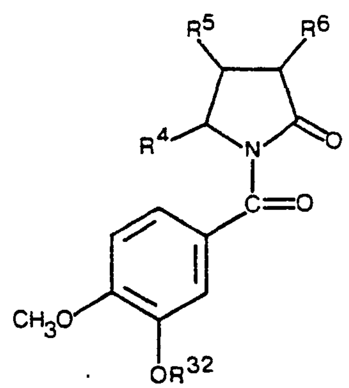

(III)

où l'un des radicaux $R^4$ et $R^6$ représente un hydrogène et l'autre forme avec $R^5$ une deuxième liaison C—C et $R^{32}$ représente un hydrogène ou un groupe protecteur séparable par réduction.

2. Procédé selon la revendication 1, caractérisé en ce qu'on prépare la (R)-1-(p-méthoxybenzoyl)-3-hydroxy-2-pyrrolidinone.

3. Procédé selon la revendication 1, caractérisé en ce qu'on prépare la (R,S)-1-(p-méthoxybenzoyl)-3-hydroxy-2-pyrrolidinone.

4. Procédé selon la revendication 1, caractérisé en ce qu'on prépare la (R,S)-1-(p-méthoxybenzoyl)-4-hydroxy-2-pyrrolidinone.

5. Procédé selon la revendication 1, caractérisé en ce qu'on prépare la 1-(3-hydroxy-4-méthoxybenzoyl)-2-pyrrolidinone.